(19) Europäisches Patentamt
European Patent Office
Office européen des brevets

(11) **EP 2 462 964 A1**

(12) **DEMANDE DE BREVET EUROPEEN**

(43) Date de publication:
**13.06.2012 Bulletin 2012/24**

(51) Int Cl.:
*A61L 31/04* *(2006.01)*    *A61L 31/14* *(2006.01)*

(21) Numéro de dépôt: **11306638.5**

(22) Date de dépôt: **12.12.2011**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **13.12.2010  FR 1060414**

(71) Demandeur: **Perouse Medical**
**60173 Ivry le Temple (FR)**

(72) Inventeurs:
• **Dao, Vithuy**
**94260 Fresnes (FR)**
• **Michelot, Robert**
**92160 Antony (FR)**
• **Perouse, Eric**
**75016 Paris (FR)**

(74) Mandataire: **Blot, Philippe Robert Emile**
**Cabinet Lavoix**
**2, place d'Estienne d'Orves**
**75441 Paris Cedex 09 (FR)**

(54) **Dispositif médical destiné à entrer en contact avec un tissu d'un patient et procédé de fabrication associé.**

(57) Ce dispositif comprend un corps (12) comportant des fibres dépourvues de cellulose oxydée non résorbables. Le corps (12) comporte également des fibres contenant de la cellulose oxydée résorbables.

FIG.1

EP 2 462 964 A1

**Description**

La présente invention concerne un dispositif médical destiné à entrer en contact avec un tissu d'un patient, comprenant un corps comportant des fibres dépourvues de cellulose oxydée.

Un tel dispositif médical est destiné à former par exemple une prothèse ou une endoprothèse vasculaire, une membrane de régénération tissulaire guidée, un tube, une plaque de contention, un cathéter de dialyse, un cathéter de perfusion, un cathéter de transfusion, un cathéter de nutrition artificielle, un implant transcutané, un treillis pour ingénierie tissulaire, un substitut osseux micro et macro poreux, un substitut de dure mère, une matrice de thérapie cellulaire, un fil de suture, un pansement à usage médical ou un patch vasculaire.

On connaît de FR 2 742 042 un dispositif médical du type précité formant une prothèse vasculaire tubulaire.

Les prothèses vasculaires sont généralement constituées d'un tissu ou d'un tricot à mailles serrées. Avant leur implantation, elles sont enduites d'un revêtement qui assure à la fois la biocompatibilité et l'étanchéité du dispositif.

Ce revêtement est par exemple formé à base de collagène, de gélatine ou d'une composition comprenant un polymère biorésorbable.

La prothèse vasculaire, une fois implantée dans l'organisme, est destinée à convoyer du sang en remplacement d'un vaisseau sanguin obstrué ou abîmé.

Pour ce faire, la couche de revêtement garantit l'étanchéité de la prothèse une fois implantée, avant que des cellules ne colonisent les parois du substrat textile pour maintenir l'étanchéité.

A cet effet, lorsque le chirurgien rétablit la pression artérielle après avoir cousu la prothèse, le revêtement de collagène empêche le sang de passer et de suinter à travers les mailles du tricot de la prothèse, de sorte que le patient ne perd pas trop de sang.

De telles prothèses donnent entière satisfaction, notamment en ce qui concerne l'étanchéité.

Néanmoins, il peut être utile dans certains cas de s'affranchir des revêtements d'origine animale, tout en conservant une bonne étanchéité et de bonnes propriétés mécaniques.

Un but de l'invention est donc d'obtenir un dispositif médical, notamment une prothèse vasculaire, qui présente une structure mécanique adéquate, tout en conservant des propriétés d'étanchéité et d'hémostaticité satisfaisantes.

A cet effet, l'invention a pour objet un dispositif médical du type précité, caractérisé en ce que le corps comporte des fibres contenant de la cellulose oxydée

Le dispositif selon l'invention peut comprendre l'une ou plusieurs des caractéristiques suivantes, prise(s) isolément ou suivant toute(s) combinaison(s) techniquement possible(s) :

- au moins une partie des fibres dépourvues de cellulose oxydée est assemblée avec les fibres contenant de la cellulose oxydée pour former au moins un fil composé, le fil composé étant tressé, tissé ou tricoté, pour former le corps ;
- les fibres contenant de la cellulose oxydée forment au moins un premier fil, les fibres dépourvues de cellulose oxydée formant au moins un deuxième fil, le premier fil et le deuxième fil étant tressés, tissés ou tricotés pour former le corps ;
- le corps est un non-tissé ;
- les fibres du corps non tissé sont orientées suivant une direction préférentielle ;
- les fibres dépourvues de cellulose oxydée sont choisies parmi les polymères synthétiques et les polymères naturels dépourvus de cellulose oxydée ;
- les fibres dépourvues de cellulose oxydée sont formées d'un polymère du groupe constitué par les polyamides, les polyoléfines, les polymères halogénés, les polyesters, les polyuréthanes, les polymères fluorés, les acides polylactiques ou polyglycidiques ou leurs mélanges ;
- la cellulose oxydée contenue dans les fibres contenant de la cellulose oxydée est fonctionnalisée par un groupe fonctionnel ;
- la cellulose oxydée comporte un groupement carboxyle porté par le carbone 6 de l'unité d'anhydroglucose, le groupement carboxyle étant fonctionnalisé par le groupe fonctionnel ;
- le groupe fonctionnel est choisi parmi un agent bioactif tel qu'un anticoagulant, un antithrombogénique, un antimitotique, un agent anti-prolifération, un agent anti-adhésion, un agent anti-migration, un promoteur d'adhésion cellulaire, un facteur de croissance, une molécule antiparasitaire, un anti-inflammatoire, un angiogénique, un inhibiteur de l'angiogénèse, une vitamine, une hormone, une protéine, un antifongique, une molécule antimicrobienne, un antiseptique, un agent réticulant, un agent de contraste ;
- le corps forme un tube ou une feuille ;
- le corps délimite une paroi étanche aux liquides, avantageusement étanche au sang ;
- le dispositif forme une prothèse ou une endoprothèse vasculaire, une membrane de régénération tissulaire guidée, un tube, une plaque de contention, un cathéter de dialyse, un cathéter de perfusion, un cathéter de transfusion, un cathéter de nutrition artificielle, un implant transcutané, un treillis pour ingénierie tissulaire, un substitut osseux micro et macro poreux, un substitut de dure mère, une matrice de thérapie cellulaire, un fil de suture, un pansement à usage médical ou un patch vasculaire ;

- le corps contient des fibres dépourvues de cellulose oxydées non résorbables, avantageusement à l'exception des fibres choisies parmi choisies parmi les polyamides, (tels que les nylons), le polyteréphtalate d'éthylène, les polyacrylates, les polyuréthanes non hydrolysables, les polyéthylènes glycol, les poly-α-oléfines du type polyéthylène et les polymères halogénés tels que les poly(halogéno-α-oléfines) comme le polytétrafluoroéthylène (PTFE) commercialisé sous le nom de Teflon ou le fluorure de polyvinylidène (PVDF), leurs copolymères ou le mélange de ces polymères.

L'invention a également pour objet un procédé de fabrication d'un dispositif médical destiné à entrer en contact avec un tissu d'un patient, caractérisé en ce qu'il comporte une étape de réalisation d'un corps comportant des fibres dépourvues de cellulose oxydée et des fibres comprenant de la cellulose oxydée.

Le procédé selon l'invention peut comprendre l'une ou plusieurs des caractéristiques suivantes, prise(s) isolément ou suivant toute(s) combinaison(s) techniquement possible(s) :

- il comporte les étapes suivantes :
- fourniture de fibres dépourvues de cellulose oxydée et de fibres comprenant de la cellulose destinée à être oxydée ;
- formation du corps à partir des fibres dépourvues de cellulose oxydée et des fibres de cellulose destinée à être oxydées ;
- oxydation sélective des fibres de cellulose destinées à être oxydées, après formation du corps pour former de la cellulose oxydée ;
- il comprend, avant la formation du corps, l'assemblage d'au moins un fil composé comportant des fibres dépourvues de cellulose oxydée et des fibres de cellulose destinée à être oxydée, et le tressage, le tricotage ou le tissage du fil composé pour former le corps ;
- la formation du corps comporte la fourniture d'au moins un premier fil formé de fibres dépourvues de cellulose oxydée et d'au moins un deuxième fil formé de fibres de cellulose destinée à être oxydée, puis le tressage, le tissage ou le tricotage du premier fil avec le deuxième fil pour former le corps ;
- il comporte, après l'étape d'oxydation, la fonctionnalisation au moins partielle de la cellulose oxydée par un groupe fonctionnel ;

L'invention sera mieux comprise à la lecture de la description qui va suivre, donnée uniquement à titre d'exemple, et faite en se référant aux dessins annexés, sur lesquels :

- la figure 1 est une vue de côté d'un premier dispositif médical selon l'invention formant une prothèse vasculaire ;
- la figure 2 est un schéma synoptique fonctionnel illustrant les différentes étapes d'un procédé de fabrication d'un dispositif médical selon l'invention ;
- la figure 3 est une figure analogue à la figure 1 d'un deuxième dispositif médical selon l'invention ;
- la figure 4 est une vue analogue à la figure 1 d'un troisième dispositif selon l'invention formant par exemple une compresse ou un patch vasculaire.

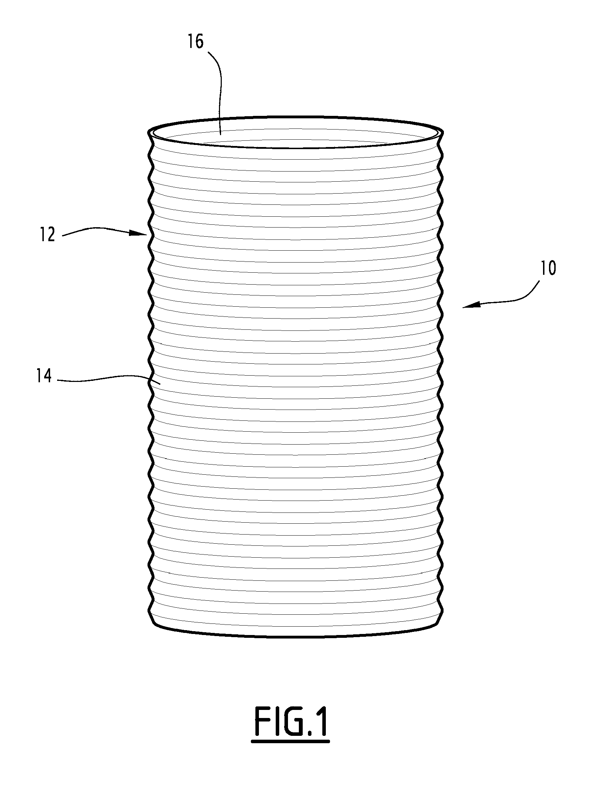

Un premier dispositif médical 10 selon l'invention est représenté sur la figure 1.

Selon l'invention, le premier dispositif médical 10 comprend un corps 12 comportant des fibres dépourvues de cellulose oxydée et des fibres contenant de la cellulose oxydée.

Dans cet exemple, le corps 12 est formé par exemple d'un tissu réalisé à base de fils tissés, tressés ou tricotés. Dans une variante qui sera détaillée plus bas, le corps 12 est un non-tissé réalisé par un assemblage de fibres liées entre elles.

Le corps 12 est un corps macroscopique, c'est-à-dire présentant au moins une dimension supérieure à 5 mm, de préférence au moins égale à 1 cm.

Les fibres contenant de la cellulose oxydée sont obtenues à partir de fibres de cellulose de formule générale -(C$_6$H$_{10}$O$_5$)n-. De manière connue, la cellulose est un homopolymère appartenant à la classe des polysaccharides. Elle est formée d'un enchaînement linéaire de molécules de glucose reliées par des liaisons glycosidiques β-1,4.

Par « cellulose oxydée », on entend, au sens de la présente invention, une cellulose dans laquelle au moins une partie des groupes alcool primaires portés par le carbone 6 de l'unité d'anhydroglucose est oxydée en un acide carboxylique éventuellement fonctionnalisé, comme on le verra plus bas.

Le degré d'oxydation de la cellulose oxydée est supérieur à 1 %. De préférence, ce degré d'oxydation est compris de 1% à 25%, préférentiellement de 5% à 20% et avantageusement de 12% à 18%.

Dans le cadre de la présente description, on entend par « degré d'oxydation » d'un matériau à base de polymère partiellement oxydé la masse des groupements acide carboxylique contenus dans 100 g dudit matériau, prise en pourcentage.

Le degré d'oxydation peut être déterminé par titrimétrie, selon la méthode d'échange de calcium (Sobue and Okubo, 1956), recommandée par United States Pharmacopeia (USP, 1990), suivant le protocole suivant :

Dans un erlen de 250 mL, introduire 0,10 g du tissu de cellulose partiellement oxydée à analyser, ajouter 50 mL d'eau purifiée et 30 mL d'une solution d'acétate de calcium à 0,5 N. Agiter le mélange sur un agitateur orbital pendant 3 heures.

Prélever 30 mL de l'échantillon et titrer avec une solution aqueuse de NaOH (0,01 N) en présence de phénolphtaléine. Le volume de NaOH consommé est corrigé en comparant ce volume avec un échantillon témoin ne contenant pas de cellulose oxydée.

La quantité équivalente de fonction acide carboxylique correspond au degré d'oxydation. Le degré d'oxydation, exprimé en %, est calculé selon la formule suivante :

$$\text{Degré d'oxydation (\%)} = \frac{8}{3} \frac{V \times N \times M_{COOH}}{m_{tissu}} \times 100$$

où :

- N (mol/L) est la normalité de la solution de NaOH (ici N = 0,01 mol/L),
- V (L) est le volume de solution de NaOH consommé pour titrer l'échantillon,
- $M_{COOH}$ (g/mol) est la masse molaire d'une fonction acide carboxylique ($M_{COOH}$ = 45 g/mol),
- $m_{tissu}$ (g) est la masse du tissu de cellulose partiellement oxydée analysé (m = 0,10 g).

Le terme « fibre » s'entend comme une formation élémentaire filamenteuse se présentant sous forme d'un faisceau. Une fibre diffère d'une mousse notamment en ce qu'elle présente une longueur supérieure à sa largeur, avantageusement une longueur supérieure à 10 fois sa largeur.

Dans le cadre de la présente description, on entend par « matériau à base de fibres solidarisées » ou « matériau fibreux », un matériau solide comprenant un ensemble de fibres liées entre elles, par exemple sous la forme d'un fil, ou sous la forme d'un tissu formé de fils tissés ou tricotés par exemple, ou bien sous la forme d'un non-tissé, ou bien encore sous la forme d'une nappe de fibres formant une matrice orientée.

Dans le cadre de la présente description, on entend par « fil » un assemblage linéaire de fibres ou de fibrilles liées entre elles de manière solidaire. Un fil de cellulose est donc à distinguer d'une fibre de cellulose, qui désigne un objet individuel et non un assemblage solidaire d'objets. Typiquement, un fil est obtenu par filage de fibres du même type, mais peut également être obtenu par filage de fibres différentes, comme par exemple des fibres de cellulose en combinaison avec des fibres synthétiques.

Le polymère constitutif des fibres de cellulose comprend des unités anhydroglucose. Il peut par exemple s'agir de fibres de cellulose, à savoir constituées par un homopolymère de glucose avec des liaisons glycosidiques β-1,4. Alternativement, il peut s'agir de cellulose modifiée, sous réserve qu'elle comporte des unités anhydroglucose, éventuellement en liaison avec d'autres unités, par exemple de la viscose qui correspond à un fil obtenu à partir de cellulose régénérée. Alternativement, il peut également s'agir de cellulose d'origine bactérienne. Avant oxydation des fibres, le degré d'oxydation du polymère constitutif des fibres est généralement inférieur à 1%.

Les fibres de cellulose sont obtenues par exemple à base de cellulose naturelle, c'est-à-dire de fibres directement issues d'un végétal, soit en étant récoltées sur le végétal, soit en étant obtenues par un traitement mécanique du végétal, comme par un broyage, un pressage, un concassage et/ou une séparation. Les fibres de cellulose sont également des fibres de cellulose modifiée, c'est-à-dire de cellulose naturelle ou de cellulose naturelle solubilisée, ayant réagi avec un composant chimique.

Le terme « fibres de cellulose » inclut aussi au sens de la présente invention, les fibres de cellulose régénérées, c'est-à-dire de cellulose naturelle, éventuellement modifiée, solubilisées dans un solvant, puis reformées sous forme de fibres.

Les fibres contenant de la cellulose contiennent au moins 1 % en moles, par exemple plus de 50% en moles de cellulose ou d'un dérivé de la cellulose.

Des exemples de fibres de cellulose naturelle d'origine végétale sont le coton, le chanvre, la jute, la laine, ou la pulpe de bois.

Les fibres de cellulose artificielle sont obtenues par un procédé de traitement de la cellulose naturelle.

Ces fibres sont par exemple définies de manière générique par le Bureau International de la Standardisation des Fibres Artificielles (BISFA) comme étant des fibres de cellulose de type « acétate », ou « tri-acétate » obtenues par acétylation des groupes hydroxyles de la cellulose, des fibres de type « alginate » obtenues à partir des sels métalliques de l'acide alginique, comme par exemple les sels alcalins ou alcalinoterreux de l'acide alginique, tels que l'alginate de calcium, ou des fibres à base de cellulose de type « cupro » obtenues par le procédé « cuprammonium », dans lequel la cellulose naturelle est dissoute dans un composé comprenant du cuivre et une amine tels que le dihydroxyde de cupratetratamine.

Avantageusement, les fibres de cellulose sont des fibres de viscose.

Par « fibre de viscose », on entend au sens de la présente invention des fibres obtenues par le procédé « viscose » selon la définition du BIFSA, dans lequel avantageusement une solution basique de xanthate de cellulose est étirée sous forme de fibres à partir d'un ou plusieurs bains de régénération. Ces fibres présentent parfois une grande résistance à la rupture et sont alors qualifiées de « modal ».

Dans certains cas, et en variante, les fibres de cellulose sont des fibres de lyocell. Par « fibre de lyocell », on entend des fibres de cellulose obtenues par un procédé de filature à partir d'un solvant organique qui comprend un mélange de produits chimiques organiques et d'eau, le terme « filature par solvant » signifiant la dissolution de la cellulose dans le solvant sans formation d'un dérivé de la cellulose.

Dans ce procédé qualifié de « lyocell » par le BIFSA, la cellulose naturelle est par exemple dissoute dans un mélange d'eau et d'un N-oxyde amine, par exemple un N oxyde d'amine tertiaire tel que le N-oxyde de N-méthylmorpholine, et est extrudée à travers un passage d'air dans un bain de précipitation pour former des fibres.

Par « fibres dépourvues de cellulose oxydée », on entend, au sens de la présente invention des fibres dépourvues de cellulose, comme des fibres synthétiques ou des fibres comprenant de la cellulose, mais dont le degré d'oxydation est inférieur à 1%.

Par « fibres synthétiques » on entend des fibres qui n'ont aucun polymère précurseur naturel, tel que les fibres obtenues par polymérisation d'un monomère synthétique, par exemple dérivé du pétrole.

Les fibres synthétiques comprennent notamment les polyamides tels que les nylons, les polyesters tels que les polytéréphtalates d'éthylène, les polyacrylates, les polyuréthanes, les acides polylactiques et polyglycoliques, les polyéthylènes gycols, les poly($\alpha$-oléfines) du type polyéthylène et les polymères halogénés tels que les poly(halogéno-$\alpha$- oléfines) comme le polytétrafluoroéthylène (PTFE) commercialisé sous le nom TEFLON® ou le fluorure de polyvinylidène, leurs copolymères ou la combinaison de ces polymères.

Avantageusement, les fibres sont des fibres de polytéréphtalate d'éthylène ou des fibres de polytétrafluoroéthylène (PTFE).

Dans un premier mode de réalisation de l'invention, les fibres dépourvues de cellulose oxydée et les fibres comprenant de la cellulose oxydée ou de la cellulose destinée à être oxydée sont assemblées entre elles pour former un fil composé, par exemple par un procédé d'ourdissage.

Le fil composé est tissé, tressé ou tricoté seul ou avec un autre fil composé, ou encore avec un fil dépourvu de fibres comprenant de la cellulose oxydée, telle qu'un fil réalisé à base de polymère synthétique comme défini plus haut, comme par exemple un fil de PET ou de PTFE.

Les fils formés à partir des fibres de cellulose oxydée et des fibres dépourvues de cellulose oxydée présentent par exemple un titre compris entre 20 dtex et 200 dtex, notamment compris entre 40 dtex et 160 dtex. Ce titre est par exemple compris entre 50 dtex et 150 dtex pour les prothèses tricotées et entre 100 et 150 dtex pour les prothèses tissées.

Dans un exemple avantageux, le fil composé comporte des fibres de cellulose oxydée et des fibres de PET ou de PTFE assemblées entre elles.

L'oxydation de la cellulose est ainsi réalisée soit avant, soit après la réalisation du fil composé.

Dans un deuxième mode de réalisation de l'invention, le corps 12 est réalisé à base d'au moins un premier fil formé exclusivement de fibres contenant de la cellulose oxydée ou de la cellulose destinée à être oxydée et à base d'au moins un deuxième fil formé exclusivement de fibres dépourvues de cellulose oxydée.

Le premier fil et le deuxième fil sont avantageusement tressés, tissés ou tricotés pour former le corps 12.

L'oxydation de la cellulose est ainsi réalisée soit avant, soit après la réalisation du corps 12.

Dans un exemple avantageux, le deuxième fil est un fil de PET ou de PTFE.

Selon une variante avantageuse de l'invention, la cellulose partiellement oxydée est fonctionnalisée par un groupe fonctionnel.

L'étape d'oxydation permet de fonctionnaliser le polymère en vue du greffage, par voie chimique, de divers radicaux susceptibles de modifier l'interaction du matériau fibreux partiellement oxydé avec son environnement biologique.

Dans un mode de réalisation, un groupe fonctionnel est lié ou remplace le groupement hydroxyle de la fonction acide carboxylique du polymère partiellement oxydé, de sorte que la cellulose partiellement oxydée est fonctionnalisée par liaison ou addition d'un groupe fonctionnel sur les fonctions acides carboxyliques présentes sur le carbone 6 de l'unité d'anhydroglucose.

Ce groupe fonctionnel est choisi avantageusement parmi un anticoagulant, un antithrombogénique, un antimitotique, un agent anti-prolifération, un agent anti-adhésion, un agent anti-migration, un promoteur d'adhésion cellulaire, un facteur de croissance, une molécule antiparasitaire, un anti-inflammatoire, un angiogénique, un inhibiteur de l'angiogénèse, une vitamine, une hormone, une protéine, un antifongique, une molécule antimicrobienne, un antiseptique, un agent réticulant, un agent de contraste. Les groupes fonctionnels sont par exemple choisis parmi ceux décrits plus haut.

Le groupe fonctionnel est par exemple greffé sur la fonction acide carboxylique par formation d'une liaison covalente entre l'un des atomes d'oxygène du groupe acide carboxylique et un radical électrophile contenant ledit groupe fonctionnel ou son précurseur. Par exemple par formation d'une fonction ester -COO(R) où R est le groupe fonctionnel, ou bien par formation d'une fonction amide -CO-NR1R2 où R1 et/ou R2 est un groupe fonctionnel.

Les groupements acide carboxylique de la cellulose oxydée sont typiquement activés avant fonctionnalisation, en utilisant un agent de couplage et/ou un auxiliaire de couplage.

Des exemples d'agents de couplage sont les carbodiimides hydrosolubles tels que le 1-éthyl-3-(3-diméthylaminopropyl) carbodiimide (EDC), le 1-éthyl-3-(3-triméthylaminopropyl) carbodiimide (ETC) et le 1-cyclohexyl-3-(2-morphilinoéthyl) carbodiimide (CMC) ainsi que leurs sels et leurs mélanges. Dans le cadre de la présente invention, l'utilisation de l'EDC est préférée.

Des exemples d'auxiliaires de couplage sont le N-hydroxy succinimide (NHS), le N-hydroxy benzotriazole (HOBt), le 3,4-dihydro-3-hydroxy-4-oxo-1,2,3-benzotriazole (HOOBt), le 1-hydroxy-7-azabenzotriazole (HAt) et le N-hydroxysulfosuccinimide (sulfo NHS) et leurs mélanges. Sans se limiter au choix du NHS, celui-ci est préféré pour une utilisation dans la présente invention.

Lorsque le groupe fonctionnel est un agent réticulant, il est lié à au moins deux fonctions acide carboxylique distinctes du polymère partiellement oxydé ou encore à des fonctions hydroxyles présentes sur le polymère partiellement oxydé.

Des exemples d'agents réticulants sont donnés dans US 6 921 819, et peuvent par exemple être choisis parmi une polyamine ou une diamine aliphatique en présence d'un réactif activateur tel que EDC, ou bien un diglycidyl éther tel que le bis-phénol diglycidyl éther, le 1,4-butanediol diglycidyl éther (BDDE), le 1,2-bis(2,3-époxypropoxy)éthylène ou le 1-(2,3-époxypropyl)-2,3-époxycyclohexane.

Le taux d'agent réticulant permet de contrôler la résorption des fibres comprenant de la cellulose partiellement oxydée dans le cas où le matériau est implanté dans l'organisme d'un patient et au contact de ses tissus.

Des exemples d'agent de contraste sont des complexes chélates de gadolinium ou des nanoparticules superparamagnétiques à base d'oxyde de fer.

Par « agent de contraste », on entend un agent susceptible d'apparaître visible, avec un contraste par rapport au reste du dispositif médical sur une image du dispositif réalisée à travers la peau du patient, notamment par radiographie, ou par résonance magnétique nucléaire.

En variante, le groupe fonctionnel est lié physiquement au groupe carboxylique du polymère partiellement oxydé, par exemple par liaison ionique lorsque le groupement fonctionnel est lié à une polyamine.

Le corps 12 formé à partir des fibres contenant de la cellulose oxydée et des fibres dépourvues de cellulose oxydée forme une paroi 14 étanche aux liquides corporels tels que le sang, avantageusement en l'absence d'un revêtement additionnel d'étanchéité.

Ainsi, la paroi 14 est hémostatique, c'est-à-dire qu'elle empêche le passage du sang lorsque la prothèse est implantée, notamment par effet dit de « clotting » ou coagulation lorsque le sang entre en contact avec la cellulose oxydée.

L'étanchéité au sang du corps 12 est par exemple déterminée par la norme ISO 7198.

En outre, les fibres contenant de la cellulose oxydée sont résorbables, une fois implantées dans l'organisme d'un patient, ce qui favorise un repeuplement par des fibroblastes, voire une régénération cellulaire d'un organe.

Dans l'exemple représenté sur la figure 1, le corps 12 du dispositif 10 est un tube comportant une paroi 14 sensiblement cylindrique.

Dans une variante, le corps 12 présente un tronçon principal cylindrique et deux bifurcations avec une forme générale de Y.

Le corps 12 délimite intérieurement un conduit 16 de circulation du sang débouchant aux extrémités de la paroi 14.

La paroi 14 empêche le passage du sang depuis le conduit 16 vers l'extérieur par la présence de fibres contenant de la cellulose oxydée et des fibres dépourvues de cellulose oxydée, notamment en l'absence de revêtement d'étanchéité.

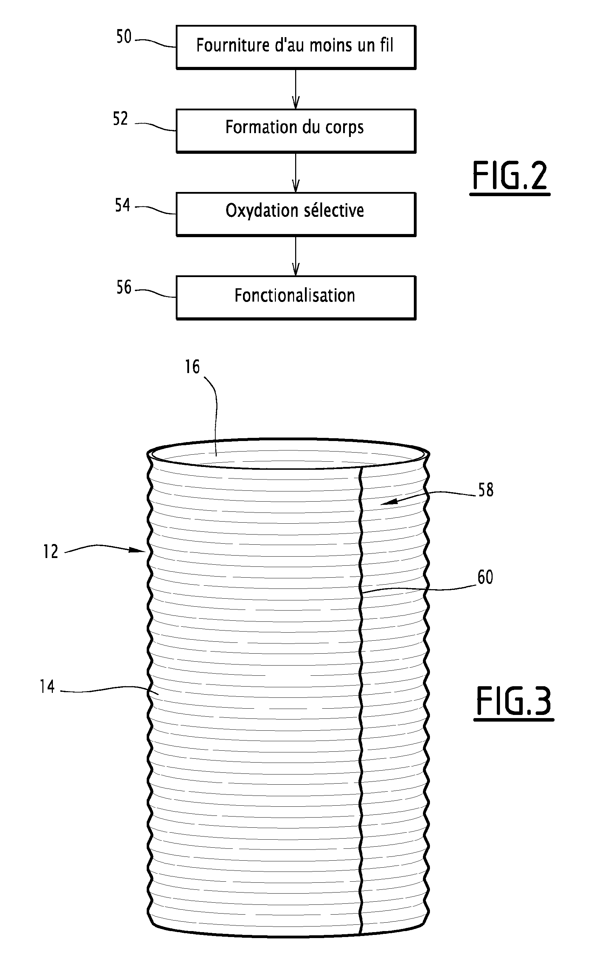

Un procédé de fabrication d'un dispositif médical 10 selon l'invention est représenté schématiquement sur la figure 2.

Ce procédé comprend une étape initiale 50 de fourniture d'au moins un fil comportant de la cellulose destinée à être oxydée, puis une étape de réalisation du corps 12, et ensuite, une étape 54 d'oxydation sélective de la cellulose destinée à être oxydée.

Le procédé comporte en outre une étape optionnelle 56 de fonctionnalisation de la cellulose oxydée.

A l'étape de fourniture 50, et dans le premier mode de réalisation décrit plus haut, au moins un fil est composé à partir de fibres de cellulose destinées à être oxydées, et de fibres dépourvues de cellulose oxydée, telles que des fibres synthétiques.

Ces fibres sont assemblées entre elles pour former un fil continu, par exemple par ourdissage.

Dans le deuxième mode de réalisation, au moins un premier fil est formé à base de fibres comprenant de la cellulose destinée à être oxydée et au moins un deuxième fil est formé à partir de fibres dépourvues de cellulose oxydée, telles que des fibres synthétiques.

Puis, à l'étape 52, le corps 12 est réalisé par entrelaçage du ou de chaque fil, par exemple par tissage, tressage ou tricotage pour former la paroi 14 comprenant des mailles. Les fibres contenant de la cellulose destinée à être oxydée ne comprenant pas de quantité significative de cellulose oxydée, elles sont particulièrement robustes. Elles peuvent être facilement tissées, tressées ou tricotées en limitant le risque de casse.

Les fibres de cellulose destinée à être oxydée ont un degré d'oxydation avantageusement inférieur à 1%. Elles sont de préférence des fibres de cellulose naturelle ou synthétique, ou bien des fibres synthétiques ou bien des fibres naturelles contenant éventuellement de la cellulose, mais dépourvues de cellulose oxydée, n'ayant pas reçu de traitement chimique oxydant particulier.

Dans un mode de réalisation privilégié, les fils sont mis sous forme de tissu à l'aide du tricotage. Différentes mailles peuvent être envisagées, notamment le jersey (classiquement tricoté avec un fil de 220 dtex à 42 filaments) ou le crochet (avantageusement tricoté avec du fil 110 dtex à 40 filaments).

Typiquement, le tissu présente un grammage de l'ordre de 50 g/m$^2$ à 100 g/m$^2$.

Puis, à l'étape 54, une oxydation sélective de la cellulose destinée à être oxydée est réalisée, sans oxydation significative des fibres dépourvues de cellulose oxydée.

Cette oxydation sélective est réalisée par exemple par un procédé de soumission de la cellulose destinée à être oxydée à l'action d'un oxyde d'azote tel que le $NO_2$ en présence de solvants. Un tel procédé implique par exemple le trempage du corps dans un bain de solvant avec barbotage de $NO_2$ gazeux, tel que décrit par exemple dans la demande de brevet EP 0 492 990.

En variante, l'oxydation sélective est réalisée par oxydation de la cellulose oxydée présente dans le corps 12 à l'aide d'un hypohalite, avantageusement en présence d'un sel d'oxoammonium, comme décrit dans la demande de brevet internationale WO 2009/016325 de la société SYMATESE. Avantageusement, l'hypohalite et l'hypochlorite de sodium et le sel d'oxoammonium est un sel de 2,2,6,6-tétraméthylpipéridine-1-oxyde (Tempo).

Dans une variante préférée, la cellulose est oxydée par mise en contact avec un mélange oxydant comprenant un hypohalite, un halite, et un sel d'oxoammonium ou un précurseur dudit sel, qui conduit à l'obtention d'un matériau à base de fibres solidarisées à base du polymère partiellement oxydé. Un tel procédé est décrit dans la demande de brevet en France de la Demanderesse déposée sous le numéro 10 60416.

L'étape d'oxydation ne modifie pas la structure macroscopique du matériau fibreux, mais seulement sa structure moléculaire. Les fibres de cellulose individuelles, c'est-à-dire des fibres non solidaires les unes des autres, sont en particulier exclues de la définition du matériau fibreux traité selon le procédé de l'invention.

Une fois l'opération d'oxydation réalisé, le degré d'oxydation des fibres de cellulose oxydée est supérieur à 1%, tel que mesuré par la méthode décrite plus haut. De préférence, ce degré d'oxydation est compris de 1% à 25%, préférentiellement de 5% à 20% et avantageusement de 12% à 18%.

Optionnellement, à l'étape 56, la cellulose oxydée est fonctionnalisée par liaison ou addition d'un groupe fonctionnel sur les fonctions acides carboxyliques présentes sur le carbone 6 de l'unité d'anhydroglucose.

Les groupes fonctionnels sont par exemple choisis parmi ceux décrits plus haut. Ces groupes sont par exemple liés de manière covalente à la cellulose oxydée par remplacement de la fonction hydroxyle de l'acide carboxylique comme décrit ci dessus.

En variante, le groupe fonctionnel est lié par liaison ionique, par interaction entre l'acide carboxylique ionisé et un ion de polarité différente.

Une fois fabriqué, le dispositif médical est avantageusement placé au contact d'un tissu d'un patient, par exemple en étant implanté dans l'organisme du patient.

Dans l'exemple représenté sur la Figure 1, du sang circule dans le conduit 16 délimité par la paroi 14, sans suinter à travers cette paroi 14 grâce à la présence des fibres de cellulose oxydée.

Une variante 58 du dispositif 10 est représentée sur la figure 3. A la différence du dispositif 10 représenté sur la figure 1, le dispositif 58 représenté sur la figure 3 comprend un corps 12 comprenant un fil de chaîne 60 comportant de la cellulose oxydée fonctionnalisée par un agent de contraste.

Le fil de chaîne 60 s'étend par exemple le long d'une génératrice de la paroi 14. Ce fil 60 est ainsi visible lorsqu'une image est réalisée à travers les tissus du patient par exemple par radiographie par rayons X ou par résonance magnétique nucléaire.

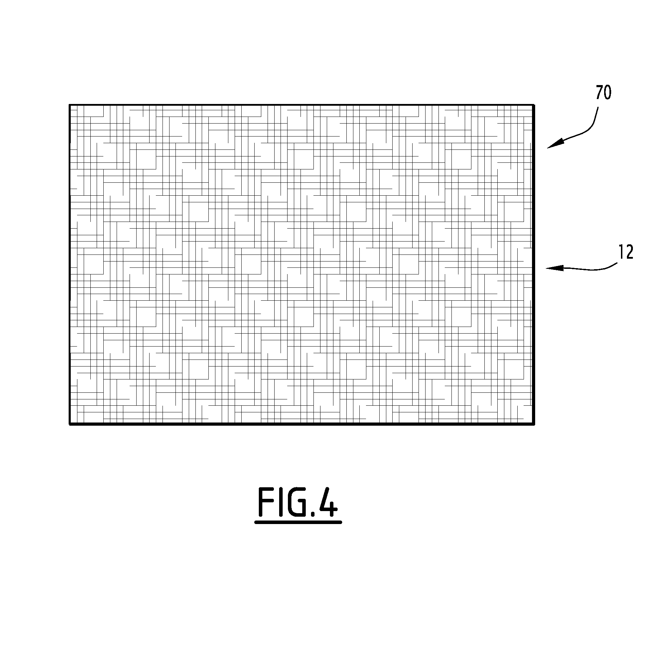

Un troisième dispositif médical 70 selon l'invention est représenté sur la figure 4. A la différence du premier dispositif, ce dispositif comporte un corps 12 en forme de feuille présentant une épaisseur inférieure à sa longueur et à sa largeur maximales.

Le corps 12 est avantageusement déformable au toucher. Ce corps 12 est par exemple une compresse ou un patch vasculaire.

Dans une variante, le corps 12 est un non-tissé. Par « non-tissé » on entend, au sens de la présente invention un substrat comprenant des fibres dépourvues de cellulose oxydée et des fibres comprenant de la cellulose oxydée dans lequel les fibres individuelles ou les filaments sont disposés de manière désordonnée dans une structure en forme de nappe et qui ne sont ni tissés, ni tricotés. Les fibres du corps non tissées sont généralement liées entre elles, soit sous l'effet d'une action mécanique, comme par exemple un jet d'eau, soit sous l'effet d'une action thermique, ou par l'ajout d'un liant.

Un tel « non tissé » est par exemple défini dans la norme ISO 9092 comme un voile ou une nappe de fibre orienté(e) directionnellement ou au hasard, lié(e) par friction et/ou cohésion et/ou adhésion, à l'exclusion du papier et des produits obtenus par tissage, tricotage, tuffetage, ou couturage.

Le corps non tissé au sens de la présente invention est obtenu par exemple par voie sèche, notamment par cardage, ou par un procédé de dépose aérodynamique désigné par le terme anglais « air laid ». En variante, le corps non tissé selon l'invention est obtenu par voie humide dans un procédé similaire à l'obtention d'un papier.

Dans une variante, les fibres destinées à former le corps non tissé sont orientées suivant une direction préférentielle avant que les fibres ne soient liées entre elles. Cette orientation préférentielle se conserve dans le corps 12 après liaison des fibres. Cette orientation des fibres favorise la colonisation cellulaire, une fois le corps 12 implanté dans le patient.

L'orientation préférentielle des fibres est obtenue par exemple en soumettant les fibres à un champ électrostatique suivant un procédé désigné par le terme anglais « electrospinning ».

Dans d'autres variantes, le dispositif forme une endoprothèse vasculaire, une membrane de régénération tissulaire guidée, un tube, une plaque de contention, un cathéter de dialyse, un cathéter de perfusion, un cathéter de transfusion, un cathéter de nutrition artificielle, un implant transcutané, un treillis pour ingénierie tissulaire, un substitut osseux micro et macro poreux, un substitut de dure mère, une matrice de thérapie cellulaire, un fil de suture, un pansement à usage médical ou encore un patch vasculaire.

Dans une variante, un groupement espaceur est inséré entre le groupe fonctionnel et le groupement carboxyle de la cellulose oxydée. Ce groupement espaceur est avantageusement labile et peut être clivé de façon sélective.

Comme il résulte directement et sans ambiguité de ce qui précède, les fibres contenant de la cellulose oxydée sont résorbables.

Le caractère résorbable de ces fibres peut être vérifié par exemple par un protocole mis en oeuvre suivant la norme NF-EN ISO 10993-6 datée de décembre 2009.

Ainsi, plusieurs éprouvettes d'essai définies suivant le paragraphe B.3 de l'annexe B de la norme précitée sont préparées à partir de l'implant. Chaque éprouvette est pesée.

La quantité totale de cellulose présente dans chaque éprouvette est mesurée par exemple par dosage. Puis, le degré d'oxydation initial de la cellulose est déterminé par la méthode décrite plus haut.

Ensuite, plusieurs éprouvettes sont implantées suivant les méthodes décrites au paragraphe 5 de la Norme précitée, par exemple dans un rat.

Puis, les éprouvettes implantées sont prélevées après une durée d'essai telle que définie dans la norme, par exemple égale à 52 semaines.

La perte de masse de chaque éprouvette est mesurée. La quantité totale de cellulose présente dans chaque éprouvette, ainsi que le dégré d'oxydation de la cellulose est mesuré comme décrit plus haut.

Une perte de masse de l'éprouvette, associée à une diminution de la quantité totale de cellulose et à une diminution du degré d'oxydation de la cellulose, indique que la cellulose oxydée présente dans l'implant s'est au moins partiellement résorbée.

Au contraire, les fibres non résorbables présentent elles une variation de masse inférieure à 10% de leur masse initiale lorsqu'elles sont implantées suivant le protocole défini ci-dessus.

Comme on l'a vu plus haut, les fibres dépourvues de cellulose oxydée non résorbables sont avantageusement choisies parmi les polyamides, (tels que les nylons), le polyteréphtalate d'éthylène, les polyacrylates, les polyuréthanes non hydrolysables, les polyéthylènes glycol, les poly-$\alpha$-oléfines du type polyéthylène et les polymères halogénés tels que les poly(halogéno-$\alpha$-oléfines) comme le polytétrafluoroéthylène (PTFE) commercialisé sous le nom de Teflon ou le fluorure de polyvinylidène (PVDF), leurs copolymères ou le mélange de ces polymères.

Plus généralement, les fibres dépourvues de cellulose oxydée non résorbables sont formées à partir de polymères et de copolymères synthétiques non biodégradables, tels que décrits dans l'ouvrage « Principles of Regenerative Medecine », 2nd édition, 2011 Elsevier, chapitre 33, pages 590 à 596.

Ces polymères incluent les polyéthylènes et leurs dérivés, notamment le polyéthylène, le polypropylène, et le polystyrène, les polymères halogénés tels que le polytetrafluoroéthylène, les polymères acryliques et méthacryliques, ainsi que les polyacrylamides, tels que le polyméthacrylate de méthyle, le poly(2-hydroxyéthyl méthacrylate), le poly(N-isopropylacrylamide), leurs copolymères ou leurs mélanges, le poly(N-isopropylacrylamide) étant avantageusement utilisé en copolymère.

En variante, les fibres dépourvues de cellulose oxydée non résorbables sont formées à partir de polyethers tels que le PEEK, ou à partir de polyéthylène glycol avantageusement utilisé en copolymère. En variante, les fibres dépourvues de cellulose oxydée non résorbables sont formées à partir d'un polymère choisi parmi les polysiloxanes ou silicones, tels que le poly(dimethylsiloxane), et leurs copolymères.

Encore en variante, les fibres dépourvues de cellulose oxydée sont formées à partir d'un polymère choisi parmi le polytéréphtalate d'éthylène, ou certains polyuréthanes stables à l'hydrolyse obtenus par exemple par réaction d'un bis chloroformate et d'une diamine ou par réaction d'un diisocyanate avec un composé dihydroxyle ou leur copolymères.

Dans une autre variante, les fibres dépourvues de cellulose oxydée non résorbables sont formées par un polymère ou un copolymère de type polyacetal comportant par exemple des motifs de type polyoxyméthylène.

## Revendications

1. Dispositif médical (10 ; 58 ; 70) destiné à entrer en contact avec un tissu d'un patient, le dispositif comprenant un corps (12) comportant des fibres dépourvues de cellulose oxydée, les fibres dépourvues de cellulose oxydée étant non résorbables, **caractérisé en ce que** le corps (12) comporte des fibres contenant de la cellulose oxydée, les fibres contenant de la cellulose oxydée étant résorbables.

2. Dispositif (10 ; 58 ; 70) selon la revendication 1, **caractérisé en ce qu'**au moins une partie des fibres dépourvues de cellulose oxydée est assemblée avec les fibres contenant de la cellulose oxydée pour former au moins un fil composé, le fil composé étant tressé, tissé ou tricoté, pour former le corps (12).

3. Dispositif (10 ; 58 ; 70) selon la revendication 1, **caractérisé en ce que** les fibres contenant de la cellulose oxydée forment au moins un premier fil, les fibres dépourvues de cellulose oxydée formant au moins un deuxième fil, le premier fil et le deuxième fil étant tressés, tissés ou tricotés pour former le corps (12).

4. Dispositif (10 ; 58 ; 70) selon la revendication 1, **caractérisé en ce que** le corps (12) est un non-tissé.

5. Dispositif (10 ; 58 ; 70) selon la revendication 4, **caractérisé en ce que** les fibres du corps non tissé sont orientées suivant une direction préférentielle.

6. Dispositif (10 ; 58 ; 70) selon l'une quelconque des revendications précédentes, **caractérisé en ce que** les fibres dépourvues de cellulose oxydée sont choisies parmi les polymères synthétiques et les polymères naturels dépourvus de cellulose oxydée.

7. Dispositif (10 ; 58 ; 70) selon la revendication 6, **caractérisé en ce que** les fibres dépourvues de cellulose oxydée sont formées d'un polymère du groupe constitué par les polyamides, les polyoléfines, les polyacrylates, les polyméthacrylates, les polyacrylamides les polymères halogénés, le polyéthylène téréphtalate, les polyuréthanes non hydrolysables, les polyethers, les polysiloxanes, les polyoxymethylènes, leurs copolymères, ou leurs mélanges.

8. Dispositif (10 ; 58 ; 70) selon l'une quelconque des revendications précédentes, **caractérisé en ce que** la cellulose oxydée contenue dans les fibres contenant de la cellulose oxydée est fonctionnalisée par un groupe fonctionnel, la cellulose oxydée comportant avantageusement un groupement carboxyle principalement porté par le carbone 6 de l'unité d'anhydroglucose, le groupement carboxyle étant fonctionnalisé par le groupe fonctionnel.

9. Dispositif (10 ; 58 ; 70) selon la revendication 8, **caractérisé en ce que** le groupe fonctionnel est choisi parmi un agent bioactif tel qu'un anticoagulant, un antitrombogénique, un antimitotique, un agent anti-prolifération, un agent anti-adhésion, un agent anti-migration, un promoteur d'adhésion cellulaire, un facteur de croissance, une molécule antiparasitaire, un anti-inflammatoire, un angiogénique, un inhibiteur de l'angiogénèse, une vitamine, une hormone, une protéine, un antifongique, une molécule antimicrobienne, un antiseptique, un agent réticulant, un agent de contraste.

10. Dispositif (10 ; 58 ; 70) selon l'une quelconque des revendications précédentes, **caractérisé en ce que** le corps (12) forme un tube ou une feuille, ou/et
délimite une paroi étanche aux liquides, avantageusement étanche au sang.

11. Dispositif selon l'une quelconque des revendications précédentes, **caractérisé en ce qu'**il forme une prothèse ou une endoprothèse vasculaire, une membrane de régénération tissulaire guidée, un tube, une plaque de contention, un cathéter de dialyse, un cathéter de perfusion, un cathéter de transfusion, un cathéter de nutrition artificielle, un implant transcutané, un treillis pour ingénierie tissulaire, un substitut osseux micro et macro poreux, un substitut de dure mère, une matrice de thérapie cellulaire, un fil de suture, un pansement à usage médical ou un patch vasculaire.

12. Procédé de fabrication d'un dispositif médical (10 ; 58 ; 70) destiné à entrer en contact avec un tissu d'un patient, **caractérisé en ce qu'**il comporte une étape de réalisation d'un corps (12) comportant des fibres dépourvues de cellulose oxydée, les fibres dépourvues de cellulose oxydée étant non résorbables et des fibres comprenant de la cellulose oxydée, les fibres contenant de la cellulose oxydée étant résorbables.

13. Procédé selon la revendication 12, **caractérisé en ce qu'**il comporte les étapes suivantes :

- fourniture de fibres dépourvues de cellulose oxydée et de fibres comprenant de la cellulose destinée à être oxydée ;
- formation du corps (12) à partir des fibres dépourvues de cellulose oxydée et des fibres de cellulose destinée à être oxydées ;
- oxydation sélective des fibres de cellulose destinées à être oxydées, après formation du corps (12) pour former de la cellulose oxydée.

14. Procédé selon la revendication 13, **caractérisé en ce qu'**il comprend, avant la formation du corps (12), l'assemblage d'au moins un fil composé comportant des fibres dépourvues de cellulose oxydée et des fibres de cellulose destinée à être oxydée, et le tressage, le tricotage ou le tissage du fil composé pour former le corps (12).

15. Procédé selon la revendication 14, **caractérisé en ce que** la formation du corps (12) comporte la fourniture d'au moins un premier fil formé de fibres dépourvues de cellulose oxydée et d'au moins un deuxième fil formé de fibres de cellulose destinée à être oxydée, puis le tressage, le tissage ou le tricotage du premier fil avec le deuxième fil pour former le corps (12).

16. Procédé selon l'une quelconque des revendications 12 à 15 **caractérisé en ce que** les fibres dépourvues de cellulose oxydée sont formées d'un polymère du groupe constitué par les polyamides, les polyoléfines, les polyacrylates, polymétacrylates, les polyacrylamides, les polymères halogénés, le polyéthylène téréphtalate, les polyuréthanes non hydrolysables, les polyethers, les polysiloxanes, les polyoxymethylènes, leurs copolymères ou leurs mélanges.

17. Procédé selon l'une quelconque des revendications 12 à 16, **caractérisé en ce qu'**il comporte, après l'étape d'oxydation, la fonctionnalisation au moins partielle de la cellulose oxydée par un groupe fonctionnel.

## FIG.1

## FIG.2

| 50 | Fourniture d'au moins un fil |
| 52 | Formation du corps |
| 54 | Oxydation sélective |
| 56 | Fonctionalisation |

## FIG.3

FIG.4

**EP 2 462 964 A1**

Europäisches
Patentamt

European
Patent Office

Office européen
des brevets

# RAPPORT DE RECHERCHE EUROPEENNE

Numéro de la demande

EP 11 30 6638

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FR 2 937 244 A1 (SOFRADIM PRODUCTION [FR]) 23 avril 2010 (2010-04-23) * page 18, ligne 1 - ligne 22 * * revendication 13 * ----- | 1-4,6-17 | INV. A61L31/04 A61L31/14 |
| A | WO 2006/044881 A2 (ETHICON INC [US]; SHETTY DHANURAJ S [US]; PENDHARKAR SANYOG MANOHAR [U) 27 avril 2006 (2006-04-27) * revendications 3,5 * ----- | 1-17 | |
| A | US 2009/156711 A1 (VAN HOLTEN ROBERT W [US] ET AL) 18 juin 2009 (2009-06-18) * alinéas [0008], [0013] - alinéa [0015] * ----- | 1-17 | |

DOMAINES TECHNIQUES RECHERCHES (IPC)

A61L

Le présent rapport a été établi pour toutes les revendications

| Lieu de la recherche | Date d'achèvement de la recherche | Examinateur |
|---|---|---|
| La Haye | 2 mars 2012 | Messemanne, Jasmine |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................................
& : membre de la même famille, document correspondant

EPO FORM 1503 03.82 (P04C02)

14

**ANNEXE AU RAPPORT DE RECHERCHE EUROPEENNE
RELATIF A LA DEMANDE DE BREVET EUROPEEN NO.**

EP 11 30 6638

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-03-2012

| Document brevet cité au rapport de recherche | | Date de publication | Membre(s) de la famille de brevet(s) | | Date de publication |
|---|---|---|---|---|---|
| FR 2937244 | A1 | 23-04-2010 | AU | 2009306022 A1 | 29-04-2010 |
| | | | CA | 2740604 A1 | 29-04-2010 |
| | | | EP | 2370023 A2 | 05-10-2011 |
| | | | FR | 2937244 A1 | 23-04-2010 |
| | | | US | 2011264237 A1 | 27-10-2011 |
| | | | WO | 2010046787 A2 | 29-04-2010 |
| WO 2006044881 | A2 | 27-04-2006 | AU | 2005295367 A1 | 27-04-2006 |
| | | | BR | PI0516220 A | 26-08-2008 |
| | | | CA | 2584717 A1 | 27-04-2006 |
| | | | CA | 2626683 A1 | 27-04-2006 |
| | | | CN | 101137402 A | 05-03-2008 |
| | | | EP | 1802358 A2 | 04-07-2007 |
| | | | EP | 2345430 A1 | 20-07-2011 |
| | | | IL | 182566 A | 28-04-2011 |
| | | | JP | 2008516819 A | 22-05-2008 |
| | | | KR | 20070084400 A | 24-08-2007 |
| | | | WO | 2006044878 A2 | 27-04-2006 |
| | | | WO | 2006044881 A2 | 27-04-2006 |
| US 2009156711 | A1 | 18-06-2009 | AU | 2008338309 A1 | 25-06-2009 |
| | | | CA | 2709464 A1 | 25-06-2009 |
| | | | CN | 101932344 A | 29-12-2010 |
| | | | EP | 2219690 A2 | 25-08-2010 |
| | | | EP | 2219691 A2 | 25-08-2010 |
| | | | JP | 2011506049 A | 03-03-2011 |
| | | | KR | 20100113515 A | 21-10-2010 |
| | | | US | 2009156711 A1 | 18-06-2009 |
| | | | WO | 2009079642 A2 | 25-06-2009 |
| | | | WO | 2009079645 A2 | 25-06-2009 |

EPO FORM P0460

Pour tout renseignement concernant cette annexe : voir Journal Officiel de l'Office européen des brevets, No.12/82

**RÉFÉRENCES CITÉES DANS LA DESCRIPTION**

**Documents brevets cités dans la description**

- FR 2742042
- US 6921819 B
- EP 0492990 A
- WO 2009016325 A

**Littérature non-brevet citée dans la description**

- Principles of Regenerative Medecine. Elsevier, 2011, 590-596